# EUROPEAN PATENT SPECIFICATION

(11) **EP 2 926 807 B1**
(45) Date of publication and mention of the grant of the patent: **09.11.2016**
(21) Application number: 14853287.2
(22) Date of filing: 24.09.2014
(51) Int. Cl.: A61K 31/047, A61K 31/045, A61K 31/375, A61P 1/00

(54) **INTESTINE CLEANSING COMPOSITION**
DARMREINIGUNGSZUSAMMENSETZUNG
COMPOSITION DE LAVAGE INTESTINAL

(30) Priority: 17.10.2013 KR 20130124072; 22.11.2013 KR 20130143118
(43) Date of publication of application: 07.10.2015
(73) Proprietor: Kang, Yoon Sik, Seocho-gu, Seoul 137-919 (KR); Shin, Chung Yoon, Seocho-gu, Seoul 137-919 (KR); Kang, Hyun Suk, Seocho-gu, Seoul 137-919 (KR); Kang, Hyun Yee, Seocho-gu, Seoul 137-919 (KR)
(72) Inventor: Kang, Yoon Sik, Seocho-gu, Seoul 137-919 (KR); Shin, Chung Yoon, Seocho-gu, Seoul 137-919 (KR); Kang, Hyun Suk, Seocho-gu, Seoul 137-919 (KR); Kang, Hyun Yee, Seocho-gu, Seoul 137-919 (KR)
(74) Representative: Gulde & Partner
(86) International application number: PCT/KR2014/008892
(87) International publication number: WO 2015/056896

(56) References cited:
- EP-A1- 2 322 190
- WO-A2-2011/027363
- KR-A- 20060 095 081
- US-A- 5 274 001
- US-A1- 2010 297 264
- US-A1- 2012 195 980
- KORSTEN M A ET AL: "A Prospective Assessment of Renal Impairment After Preparation for Colonoscopy: Oral Sodium Phosphate Appears to Be Safe in Well-Hydrated Subjects with Normal Renal Status", DIGESTIVE DISEASES AND SCIENCES, KLUWER ACADEMIC PUBLISHERS-PLENUM PUBLISHERS, NE, vol. 55, no. 7, 16 October 2009 (2009-10-16), pages 2021-2029, XP019816464, ISSN: 1573-2568

## Description

### FIELD OF THE INVENTION

The present invention relates to a bowel cleansing preparation. More particularly, the present invention relates to a novel bowel cleansing preparation comprising high concentrations of a specific sugar alcohol and ascorbic acid so as to improve the ease of administration by reducing the total dosage and yet improve bowel cleansing efficacy while ensuring safety.

### BACKGROUND OF THE INVENTION

According to the reports from the World Cancer Research Fund International, colon cancer is the third most common cancer worldwide with incidences of colon cancer during the year 2012 estimated to be 1.4 million worldwide and expected to be 2.4 million in the year 2035. For the diagnosis of colon cancer, colonoscopy is essential. Moreover, colonoscopy is very important because colon cancer can be prevented if colon polyps, which are considered to be the source of colon cancer, are detected during colonoscopy and removed.

For accurate colonoscopy examination, bowel cleansing prior to the procedure is essential; since stool may be retained inside the colon even after fasting for days, it is necessary to cleanse the colon thoroughly by artificial methods prior to the examination. For this purpose, methods have been developed where cleansing preparations comprised of laxative agents are ingested to empty the colon. An ideal bowel cleansing preparation should have high cleansing power, ease of administration, as well as safety.

Firstly, high cleansing efficacy is an essential requirement for an accurate colonoscopy procedure. If too much residue remains after bowel cleansing, it is difficult to perform an accurate examination, and the poor visualization increases the risk of complications such as perforation, and sometimes the painful bowel preparation has to be repeated, which might result in the patient's refusal or avoidance of the examination. Thus, reliable cleansing efficacy is the most important requirement.

Secondly, the ease of administration is determined by the volume of the bowel cleansing preparation to be ingested, the taste of the preparation, and the occurrence of discomforts such as nausea, vomiting, and abdominal pain following the ingestion of the preparation. Even if a bowel cleansing preparation has excellent cleansing power, if its administration is difficult, one cannot successfully complete the intake process in accordance with the dosing regimen, resulting in inadequate bowel cleansing. In addition, the unpleasant experience with bowel cleansing often leads to the refusal of repeat examination, and rumors about the difficulties of administration may impede the popularization of colonoscopies harming public health.

Thirdly, in terms of safety, bowel cleansing should minimize temporary disorders, should not pose risks such as the production of combustible gases that can complicate operations, and should not cause irreversible chronic disorders.

Early regimens involved ingesting a large volume of up to 7-12 L of solutions like saline solutions as bowel cleansing preparations. However, administration of such cleansing preparations was accompanied by a great deal of pain and difficulties.

Later, bowel cleansing preparations using 2 L of 10% mannitol, a sugar alcohol, was introduced and garnered much expectation. However, colonic gas explosions during colonic surgery or therapeutic colonoscopy after mannitol preparation have been reported and the use of mannitol is now avoided. Bowel cleansing preparations with sorbitol have also been considered to be inappropriate in most advanced countries including the U.S. and Europe due to similar risks.

In the 1980s, bowel cleansing preparations comprising the ingestion of polyethylene glycol (PEG) formulated into a 4 L solution were developed. PEG formulations are still the most commonly used bowel cleansing preparations worldwide.

However, studies show that the percentage of bowel preparations with a PEG formulation achieving the cleansed level adequate for colonoscopy is approximately 70%. Such inadequate cleansing by PEG formulations has been demonstrated by a number of published studies. For instance, a study has reported that colonoscopy was hindered by the residual solid stool in the colon in 47.1 % of patients who had the bowel preparation with the PEG formulation "Colyte" <"Comparison between Conventional 4 L Polyethylene Glycol and Combination of 2 L Polyethylene Glycol and Sodium Phosphate Solution as Colonoscopy Preparation", Seoul National University Bundang Hospital, Department of Internal Medicine, Seoul National University College of Medicine and Liver Research Institute, Jung Won Lee et al., Korean J Gastroenterology, 56, 299-306, 2010>.

Problems with PEG formulations that are more serious than the unsatisfactory bowel cleansing efficacy include the very large volume (up to 4 L) of the formulations to be ingested, and the difficulty of ingestion due to the unpleasant taste of the PEG formulations. Such difficulty of ingestion gave rise to the wide-spread negative perception of bowel preparation, which has lead people to fear or avoid colonoscopies.

Various attempts have been made to reduce the volume of the PEG formulations to be ingested. For example, the required volume of the PEG solution may be reduced to 2 L by including a stimulant laxative such as bisacodyl tablets in the regimen such that it is administered 6-12 hours prior to the ingestion of the PEG solution, or using a magnesium citrate (Trade name Citromag^{®}) solution in combination, or adding ascorbic acid (Trade name MoviPrep^{®}). Even in such cases, it is recommended that the subject additionally drink 1 L of water after consuming the 2 L PEG solution, with the total volume of the ingested fluid amounting to 3 L.

In the case of MoviPrep^{®} type bowel preparations, ascorbic acid and sodium ascorbate are added to make up for the anticipated reduction in cleansing efficacy due to the reduced volume of fluid to be ingested. However, as the total amount of PEG contained in these bowel cleansing preparations is 200 g, the PEG content has decreased by only 15.3%, compared to the 236 g contained in the COLYTE powder which is to be administered in a 4 L volume. Thus, despite the decrease in the total volume of fluid to be ingested, complaints of patient discomfort are more common than in the case of the COLYTE powder 4 L regimen because PEG is concentrated 1.7-fold.

Although research has been indicating that phosphate preparations are less difficult to ingest and their cleansing efficacy also compares similarly or favorably to the PEG formulations, persistent concerns regarding the risk of renal injury have been a big obstacle for their utilization. The U.S. Food and Drug Administration (FDA) has withdrawn its approval of some phosphate preparations for reasons of possible renal injury. A big advantage of picosulfate preparations is that they have greatly improved taste and thus, unlike the case of PEG preparations, their consumption causes little discomfort due to unpleasant taste. For that reason, picosulfate preparations are known to be the most commonly used preparation in Europe. However, the biggest problem with picosulfate preparations lies in its relatively poor bowel cleansing efficacy. Partly because of this problem, picosulfate preparations are not widely used in the U.S. and Canada. Even in Europe where they are widely used, it is common that rather than being used by itself, picosulfate is combined with magnesium citrate to supplement the insufficient cleansing efficacy.

Since 2012, a picosulfate preparation containing magnesium citrate (PICOLIGHT^{®}), which has the same composition as Prepopik^{®} approved by the U.S. FDA has been marketed and used in Korea. However, the preparation has a problem in that the total volume of ingestion amounts to 3.45 L according to its recommended dosing instruction and its cleansing efficacy is relatively poor despite the incorporation of magnesium citrate.

As shown above, known bowel cleansing preparations have serious problems such as excessively large volumes of ingestion, difficulties in administration due to unpalatable taste, risks of substantial adverse effects such as renal disorders, causing the production of combustible colonic gases, and poor cleansing efficacy which is an essential element of a bowel cleansing preparation.

Attempts to overcome the above limitations of known bowel cleansing preparations have not been successful and, consequently, there is a strong need for the development of a novel bowel cleansing preparation that has reliable cleansing power, ease of administration, minimal side effects, and is safe.

### SUMMARY OF THE INVENTION

In an attempt to overcome the above problems in the prior art, it is an object of the present invention to provide a novel bowel cleansing preparation that is equipped not only with excellent cleansing efficacy to greatly improve the proportion of adequate bowel preparation that is still at an average of about 70% but also with ease of administration owing to the improved taste as well as safety for the human body.

It is another object of the present invention to provide a novel bowel cleansing preparation which can greatly enhance the ease of administration by reducing the required volume, which typically is up to 3-4 L, to 1 L or less and minimize discomforts such as nausea, vomiting, abdominal pain, and bloating.

It is a further object of the present invention to provide a novel bowel cleansing preparation that allows for economical mass production and commercialization.

The bowel cleansing preparation according to the present invention comprises as the first cleansing ingredient, at least one sugar alcohol selected from xylitol, sorbitol, glycerol, erythritol, threitol, arabitol, ribitol, galactitol, fucitol, iditol, inositol, volemitol, isomalt, maltitol, lactitol, maltotriitol, maltotetraitol, and polyglycitol; as the second cleansing ingredient, ascorbic acid or a mixture of ascorbic acid and a salt of ascorbic acid; and an aqueous solvent, wherein the concentration of the first cleansing ingredient is 10 g/L to 500 g/L based on the total preparation, the concentration of the second cleansing ingredient is 15 g/L to 500 g/L based on the total preparation, and the volume of the aqueous solvent is 0.1 L to 1.0 L.

By combining specific concentrations of a sugar alcohol and ascorbic acid, the bowel cleansing preparation of the present invention displays superior cleansing efficacy despite a greatly reduced volume being consumed compared with existing bowel cleansing preparations. The bowel cleansing preparation of the present invention also greatly enhances the ease of administration and compliance by significantly reducing the volume of ingestion. In addition, the bowel cleansing preparation of the present invention improves patient discomfort by causing no or minimal discomforts such as nausea, abdominal pain and vomiting during its consumption, and is safe in that it does not cause severe adverse effects such as chronic disorders or electrolyte abnormalities.

In addition, by combining specific concentrations of a sugar alcohol and ascorbic acid, the present invention achieves superior cleansing efficacy and antibacterial effect relative to the volume of the consumed fluid, eliminating the risk of production of combustible gases in the colon after bowel cleansing preparation ensuring safety in the human body.

The most stressful part about the bowel cleansing preparation procedure from the viewpoint of the patients includes large volumes of ingestion, unpleasant taste, discomforts such as abdominal pain and vomiting. It is highly significant that the present invention has solved such problems to eliminate most of the patient discomfort during the preparation, and yet enhanced the cleansing efficacy. Further, by combining a certain sugar alcohol and ascorbic acid, the present invention eliminates the risk of production of combustible gases in the colon as much as possible.

As described above, the bowel cleansing preparation of the present invention can make the bowel preparation procedure significantly easier and safer, thereby greatly contributing to the popularization of colonoscopy and, as a result, maximizing the early detection of colon cancer as well as the prevention of colon cancer by polypectomy. The bowel cleansing preparation of the present invention may also be utilized in treating and alleviating constipation.

The preparation process of the present invention allows for economical mass production and commercialization of the bowel cleansing preparation.

### BRIEF DESCRIPTION OF THE DRAWINGS

Fig. 1 is a photograph showing the results of culturing intestinal bacteria after treatment with the bowel cleansing preparations from one Example of the present invention and the Reference Examples.
Fig. 2 is a photograph of the bowel cleansing preparations of the Comparative Examples before mixing the cleansing ingredients with the solvent and those of the Examples of the present invention after mixing the cleansing ingredients with the solvent, which shows the volumes of ingestion for the preparations of the Examples of the present invention and the Comparative Examples.
Fig. 3 shows photographs representing the cleanliness of the colon following the consumption of the bowel cleansing preparations from the Examples of the present invention and the Comparative Examples.
Fig. 4a and Fig. 4b show graphs representing the rates of effective and insufficient bowel preparation following the consumption of the bowel cleansing preparations from the Examples of the present invention and the Comparative Examples.
Fig. 5 is a photograph showing a substantial amount of intracolonic bubbles generated after consumption of a bowel cleansing preparation prepared according to prior art.

### DETAILED DESCRIPTION OF THE INVENTION

The present invention will now be described in detail with reference to the drawings.

The bowel cleansing preparation of the present invention comprises, as the first cleansing ingredient, at least one sugar alcohol selected from xylitol, sorbitol, glycerol, erythritol, threitol, arabitol, ribitol, galactitol, fucitol, iditol, inositol, volemitol, isomalt, maltitol, lactitol, maltotriitol, maltotetraitol, and polyglycitol; as the second cleansing ingredient, ascorbic acid or a mixture of ascorbic acid and a salt of ascorbic acid; and an aqueous solvent, and the concentration of the first cleansing ingredient is 10 g/L to 500 g/L based on the total preparation, the concentration of the second cleansing ingredient is 15 g/L to 500 g/L based on the total preparation, and the volume of said aqueous solvent is 0.1 L to 1.0 L.

Sugar alcohols used in the present invention as the first cleansing ingredient are a cleansing ingredient that works as a laxative when used at a high concentration. The sugar alcohol used as the first cleansing ingredient for the present invention is one or more selected from xylitol, sorbitol, glycerol, erythritol, threitol, arabitol, ribitol, galactitol, fucitol, iditol, inositol, volemitol, isomalt, maltitol, lactitol, maltotriitol, maltotetraitol, and polyglycitol. In one aspect of the present invention, sugar alcohol used as the first cleansing ingredient may include xylitol or sorbitol or a mixture of xylitol and sorbitol.

With respect to the safety of sugar alcohols, along with mannitol, sorbitol has been deemed as a substance that should not be used for bowel preparation after an incidence of colonic gas explosion during a colopolypectomy procedure following bowel preparation using sorbitol was reported.

However, a review article by Ladas et al. shows that such incidence of explosion is not limited to mannitol or sorbitol, but occurs also after bowel preparation with a PEG formulation or a phosphate formulation. According to Ladas et al., a total of 20 gas explosions associated with bowel preparations were reported between 1952 and 2006, including four explosions during colopolypectomy of which two involved bowel preparation with PEG formulations, one with a phosphate formulation and another with mannitol <"Colonic gas explosion during therapeutic colonoscopy with electrocautery", Ladas SD, Karamanolis G, Ben-Soussan E., World J Gastroenterol, 13, 5295-8, 2007>.

Colonic gas explosions during surgery or therapeutic endoscopy after bowel preparation are believed to be caused by the combustible gases hydrogen and methane produced by the fermentation of sugar alcohols such as mannitol and sorbitol by intestinal bacteria. The explosive ranges for methane and hydrogen are 5-15 vol% and 4-75 vol%, respectively.

However, it has been demonstrated that hydrogen and methane gases are more likely to be produced when there are residues in the colon due to inadequate cleansing. It has been known that colonic gas explosions occurred not only with mannitol or sorbitol, but also after bowel preparation with formulations of PEG, which is another sugar alcohol, or phosphate, which is not a sugar alcohol. These facts indicate that the cause of colonic gas explosion cannot be limited to the use of particular colon cleansing preparations. Rather, the quality of colon cleansing has a significant effect in this regard. This is because when inadequate bowel cleansing leaves a lot of intestinal bacteria behind, sugar alcohols in the residual stool and the bowel cleansing solution are excessively degraded, producing hydrogen and methane in explosive concentrations.

Indeed, Nunes et al. reported that the cleansing efficacy of mannitol is inferior to PEG formulations, with the rate of adequate bowel cleansing being only 75% for mannitol as compared with 90% for PEG formulations when evaluated according to a scale proposed by the authors <"Comparative evaluation of bowel preparation for colonoscopy using mannitol and polyethylene glycol - a prospective study", Nunes BL, Belo SG, Pessoa MH, Lins Neto MA., Rev Bras Coloproctol, 28, 294-8, 2008>.

Given these observations, previously reported gas explosions after bowel preparation with mannitol or sorbitol are believed to have been affected by inadequate bowel cleansing.

However, by using a sugar alcohol as the first cleansing ingredient in combination with ascorbic acid as the second cleansing ingredient in specific concentrations, the present invention achieves superior cleansing efficacy and antibacterial effect relative to the volume of consumed fluid, eliminating the risk of explosion arising from inadequate bowel preparation ensuring safety.

The concentration of the first cleansing ingredient used in the present invention ranges from 10 g/L to 500 g/L, 20 g/L to 200 g/L, or 50g/L to 150g/L based on the total preparation, without limitation. If the concentration of the first cleansing ingredient is higher than the above range, production of the combustible gases hydrogen and methane may increase. If the concentration of the first cleansing ingredient is lower than the above range, it may lead to incomplete bowel cleansing.

Ascorbic acid, which is used as the second cleansing ingredient in the present invention, is a water-soluble vitamin known as vitamin C, an essential vitamin playing various roles in maintaining health. With an increasing interest in ascorbic acid, a variety of specialty beverages containing ascorbic acid are being marketed. These specialty beverages are manufactured to contain ascorbic acid at concentrations up to 10 mg/mL (i.e., 10 g/L).

In the present invention, however, ascorbic acid is the main cleansing ingredient which is used in a high concentration to work as a laxative and its antibacterial activity inhibits intestinal bacteria such that the production of colonic gases is minimized. For this purpose, the second cleansing ingredient is incorporated at a high concentration of 15 g/L to 500 g/L, 20 g/L to 300 g/L, or 30 g/L to 200 g/L based on the total preparation. If the concentration of the second cleansing ingredient is higher than the above range, the excessively high acidity of the preparation may lead to excessive sour taste, making it difficult to consume the preparation, and may severely stimulate the stomach to cause nausea, retching, vomiting, and heartburn during the consumption of the preparation. On the other hand, if the concentration of the second cleansing ingredient is lower than the above range, the reduced antibacterial effect may lead to the excessive production of combustible gases in the colon.

Bowel cleansing preparations of the type like MoviPrep^{®}, which is currently being marketed abroad, use PEG as the main ingredient and provide pouches containing 10.6 g of ascorbic acid powder. With these products, the instructions state that a diluted cleansing solution is prepared by dissolving 100 g of PEG in 1 L of water followed by 10.6 g of ascorbic acid powder, with the preparation and ingestion of the diluted cleansing solution repeated once again to make the total volume of ingestion 2 L. Accordingly, the concentration of ascorbic acid used in these MoviPrep^{®} or CoolPrep^{®} products is 10.6 mg/mL(i.e., 10.6 g/L), which is about the same as the highest concentration of ascorbic acid in specialty beverages and significantly less than the concentration required for the second cleansing ingredient incorporated in the present invention. Thus, there has been no known products or research articles or attempts to utilize ascorbic acid as the main cleansing ingredient by using it at a high concentration to maximize its function as a laxative. In addition, there has been no known products or research articles or attempts to inhibit the production of colonic gases by using high concentrations of ascorbic acid to utilize its antibacterial effect against intestinal bacteria.

A salt of ascorbic acid used as the second cleansing ingredient in the present invention refers to an alkaline metal or alkaline earth metal salt of ascorbic acid. Examples of the alkaline metal include, but are not limited to, sodium or potassium, and examples of the alkaline earth metal include, but are not limited to, calcium or magnesium. The salt of ascorbic acid used in the present invention may be sodium ascorbate or calcium ascorbate.

A salt of ascorbic acid reduces the acidity of a solution compared to ascorbic acid alone, enhancing the bowel cleansing efficacy by inhibiting the absorption of ascorbic acid which is known to increase with acidity.

When the second cleansing ingredient is a mixture of ascorbic acid and a salt of ascorbic acid, the mass ratio of ascorbic acid to the salt of ascorbic acid may range from 99:1 to 60:40. If the mass ratio of ascorbic acid to the salt of ascorbic acid is outside the above range, excessive amounts of sodium, calcium, potassium or magnesium may be absorbed to the body, causing electrolyte abnormalities or resulting in hypercalcemia or hypermagnesemia.

Bisacodyl, which can be used as the third cleansing ingredient in the present invention, is widely used as a stimulant laxative. While bisacodyl is usually administered once or twice daily at a dose of 5 mg to 10 mg at one time, it is possible to take up to 30 mg at one time. Bisacodyl is typically prepared and used in the form of tablets, and suspensions of bisacodyl have not been known to be prepared or used. Some of the bowel cleansing preparations using PEG as the main ingredient are available in the form of bowel prep kit products, aimed at reducing the volume of the PEG solution to be ingested by providing bisacodyl tablets in separate packages. However, because it takes 6-12 hours before the bisacodyl tablets to exhibit an effect, they should be administered 6-12 hours prior to taking a PEG bowel cleansing preparation. Thus, bisacodyl tablets are taken on the evening before the scheduled colonoscopy to induce bowel movement before administering PEG. However, such a split dose regimen makes bowel preparation take longer, adding to patient discomfort.

In the present invention, however, bisacodyl as the third cleansing ingredient is incorporated, in the form of a suspension, into the bowel cleansing preparation, thereby allowing it to exhibit an effect within 1-2 hours from ingestion and consequently augmenting the colon cleansing effect despite simultaneous administration. Moreover, as a stimulant laxative, bisacodyl promotes intestinal peristalsis to help excrete excess water remaining in the colon immediately after bowel preparation, thereby further enhancing the accuracy and smooth progress of colonoscopy. The concentration of bisacodyl as the third cleansing ingredient used in the present invention may be 5 mg/L to 50 mg/L, 10 mg/L to 40 mg/L, or 15 mg/L to 30 mg/L based on the total preparation. The total amount of bisacodyl used in the present invention may exceed the usual maximum dose of 30 mg because most of the bisacodyl administered in a suspension form is excreted without having directly contacted the intestinal mucosa.

The bowel cleansing preparation of the present invention may additionally comprise an anti-foaming agent in order to remove colonic gases or bubbles frequently seen after bowel preparation. In one aspect of the present invention, examples of suitable anti-foaming agent includes, without limitation, simethicone. When used as an anti-foaming agent in the present invention, simethicone can be incorporated, in the form of a suspension, into the bowel cleansing preparation.

In general, if a substantial amount of bubbles appear during screening colonoscopy, a diluted simethicone solution is prepared and injected into the colon through a small channel in the scope. Another approach involves separate administration of a separately provided simethicone preparation at the last stage of bowel preparation. However, when diluted simethicone is injected through a channel in the scope during the procedure, the area that can be covered by a single application of diluted simethicone is very limited. Thus, if the bubbles are formed across a large area, inconvenience arises as the application of diluted solution may need to be repeated as many as ten times or more. Even when the simethicone preparation is separately administered at the last stage of bowel preparation, the volume of the preparation is typically as small as 10 ml, which may be insufficient to prevent the formation of bubbles throughout the colon.

To address this problem, the present invention incorporates a diluted anti-foaming agent such as simethicone into the bowel cleansing preparation so that foaming is very effectively inhibited across the regions of colon reached by the bowel cleansing liquid even with a small volume of the anti-foaming agent.

The concentration of the anti-foaming agent used in the present invention may be 100 mg/L to 2 g/L, 150 mg/L to 1.5 g/L, or 200 mg/L to 1 g/L based on the total preparation in the case of simethicone, but is not limited to the above ranges in the case of anti-foaming agents other than simethicone. If the concentration of the anti-foaming agent exceeds the upper limit of the above range, it may cause adverse effects such as abdominal pain, rash, swelling of the face or tongue, breathing difficulties, etc.

Aqueous solvents that can be used in the present invention include water, carbonated water, alkaline ionized water, and beverages, for example. There is no particular limitation on the kind of beverage as long as the effect of the present invention can be achieved. Examples of beverages include coffee, various juices, cola, clear sodas, and gin and tonic. In one aspect of the present invention, carbonated water or alkaline ionized water can be used as the aqueous solvent. Working independently as a laxative, carbonated water is known to be effective in improving constipation in elderly people suffering from chronic constipation. Alkaline ionized water may increase the cleansing efficacy by partially neutralizing the acidity of the bowel cleansing preparation of the present invention, leading to reduced absorption of ascorbic acid.

The volume of the aqueous solvent can be, without limitation, 0.1 L to 1.0 L, 0.1 L to 0.9 L, 0.2 L to 0.8 L, or 0.3 L to 0.7 L. If the volume of the solvent is less than 0.1 L, ingredients may not be completely dissolved in the solvent. If the volume of the solvent is greater than 1.0 L, the large volume may lead to reduced compliance.

The bowel cleansing preparation of the present invention may further comprise known cleansing ingredients or adjuncts for augmenting the cleansing effect. Examples of additional cleansing ingredients and adjuncts include citric acid, magnesium component such as magnesium oxide, docusate sodium, senna extract (e.g., sennoside), aloe extract (e.g., aloin), etc. and pectin, zinc component such as zinc oxide, caffeine, etc.

Citric acid is a natural substance present in citrus fruits in high concentrations. It makes sour taste and is used as a preservative in a variety of beverages and food. Citric acid is classified as a very safe substance, and the FDA and the FAO/WHO have recognized that the ingestion of excessive amounts of citric acid causes little harm. In the bowel cleansing preparation of the present invention, citric acid can be incorporated as an additional cleansing ingredient working as a laxative. In this case, the concentration of citric acid may be 1 g/L to 15 g/L based on the total preparation. If its concentration exceeds the above range, patient compliance may be reduced due to heartburn and strong sour taste.

Magnesium is also a substance than can function as a laxative. However, it should be used with caution in people with renal disorders because administering an excess amount greater than 3 g at one time may cause adverse effects from hypermagnesemia.

Pectin and zinc can enhance the laxative effect of ascorbic acid by inhibiting its gastrointestinal absorption. Pectin can be used at a concentration of 0.1 g/L to 2 g/L based on the total preparation, and may not be dissolved if used at a concentration greater than the above range. Zinc oxide can be used at a concentration of 10 mg/L to 400 mg/L based on the total preparation, and its excessive intake beyond the above range may cause vomiting, retching, etc.

Caffeine may be used to fight the sluggishness that may be felt during bowel preparation and enhance the cleansing efficacy. Up to 30 mg of caffeine can be used based on the total preparation. Excessive intake of caffeine beyond the above range can cause tachycardia, anxiety, sleep disturbance, etc.

Substances that may be used as adjunctive laxatives also include docusate sodium (10 mg/L to 400 mg/L. Excessive intake can cause vomiting, abdominal pain, etc.), senna extract (sennoside, 10 mg/L to 50 mg/L, Excessive intake can cause vomiting, abdominal pain, etc.), aloe extract (aloin, 10 mg/L to 50 mg/L. Prolonged excessive intake can cause reduced bowel function), and the like.

The bowel cleansing preparation of the present invention may additionally comprise ingredients for improving compliance and alleviating upper gastrointestinal symptoms such as nausea, retching and vomiting that are frequently observed during bowel preparation.

Because the bowel cleansing preparation of the present invention containing a large amount of ascorbic acid and citric acid is high in acidity and may be difficult to consume due to excessive sour taste, it may comprise a bicarbonate salt such as sodium bicarbonate and potassium bicarbonate to neutralize the acidity.

Sodium bicarbonate can be used at a concentration ranging from 0.1 g/L to 10 g/L based on the total preparation. If used beyond the above range, it may lead to reduced compliance due to excessive saltiness and cause hypernatremia. Potassium bicarbonate can be used at a concentration ranging from 0.1 g/L to 20 g/L based on the total preparation. If used beyond the above range, it can cause hyperkalemia due to the intake of potassium exceeding its daily recommended dose. Hyperkalemia poses the risk of heart disorders, for example.

The bowel cleansing preparation of the present invention may further comprise an extract, powder or concentrate of ginger, peppermint, chamomile or the like in the form of aqueous solution for the purpose of enhancing the compliance by alleviating symptoms such as nausea, vomiting and abdominal pain. These ingredients work as gastrointestinal soother and can be incorporated at a concentration of 5 g/L to 50 g/L based on the total bowel cleansing preparation. If used excessively beyond the above range, such gastrointestinal soothers may reduce the cleansing efficacy by inhibiting bowel movement.

The bowel cleansing preparation of the present invention may include sweeteners to improve taste. Examples of suitable sweeteners include, without limitation, sucralose, maltodextrin, glucose, sucrose, dextrose, saccharin, aspartame, and stevia. Such sweeteners can be used in a range from 0.01 mg/L to 10 g/L based on the total bowel cleansing preparation. Using an excessive amount beyond the above range may cause discomforts such as nausea and retching.

The bowel cleansing preparation of the present invention may additionally include suitable amounts of edible fruit flavorings to enhance compliance. Such fruit flavorings may be strawberry flavor, orange flavor, apple flavor, grape flavor, lemon flavor, banana flavor, cherry flavor, etc.

The bowel cleansing preparation of the present invention may additionally comprise a suitable amount of antioxidant to prevent the oxidation of ascorbic acid. Such antioxidant includes, without limitation, ferulic acid, amino acids such as glycine and histidine, hyaluronic acid, and tocopherol.

In addition, the bowel cleansing preparation of the present invention may additionally comprise a chelating agent to chelate the trace amounts of iron and copper ions potentially contained in the aqueous solvent. Examples of such chelating agents include, without limitation, the Versene™ CA chelating agent (The Dow Chemical Company).

The bowel cleansing preparation of the present invention can be a one-part product or a two-part product.

In one aspect of the present invention, the bowel cleansing preparation can be formulated into a one-part product in which the entire ingredients including the first cleansing ingredient and the second cleansing ingredient and, as necessary, the third cleansing ingredient, anti-foaming agent, gastrointestinal soother, and other additives are packaged in a container together with the aqueous solvent.

In another aspect of the present invention, the bowel cleansing preparation can be packaged into a two-part product. For example, the first cleansing ingredient and second cleansing ingredient may be packaged together in the same container while the aqueous solvent is separately packaged; the second cleansing ingredient and the aqueous solvent may be packaged together in the same container while the first cleansing ingredient is separately packaged; the first cleansing ingredient and the aqueous solvent may be packaged together in the same container while the second cleansing ingredient is separately packaged; the first cleansing ingredient and part of the aqueous solvent may be packaged together in the same container while the second cleansing ingredient and part of the aqueous solvent are packaged together in the same container; or the first cleansing ingredient and second cleansing ingredient may be packaged in the same container together with part of the aqueous solvent while the remaining part of the aqueous solvent is separately packaged. In addition, the third cleansing ingredient, anti-foaming agent, gastrointestinal soother, and other additives can be incorporated into the two-part product, as necessary, in various combinations.

A method of formulating the bowel cleansing preparation of the present invention into a two-part product that is packaged in the form of a highly concentrated solution and a vehicle and using the product is now explained. For example, the entire ingredients including the first cleansing ingredient and second cleansing ingredient and, as necessary, the third cleansing ingredient, anti-foaming agent, gastrointestinal soother, and other additives are dissolved in a minimal amount of an aqueous solvent to give a highly concentrated solution, and users may dilute the solution in a beverage (making up the remaining volume as an aqueous solvent) they have chosen as vehicle. In such a case, the volume of the aqueous solvent for forming the highly concentrated solution may be 0.05 L to 0.2 L, and the amount of the beverage of the user's choice, i.e., vehicle may be the remaining volume for an aqueous solvent. If necessary, each solid ingredient of the composition is separately packaged as well as the aqueous solvent, and users can mix the entire ingredients at the time of ingestion. Various other forms of packaging can also be used and the form of the two-part product is not limited by the packaging methods described above.

In manufacturing the bowel cleansing preparation of the present invention, the entire ingredients including the first cleansing ingredient and second cleansing ingredient, and, as necessary, the third cleansing ingredient, anti-foaming agent, gastrointestinal soother, and other additives can be mixed with the aqueous solvent at the same time, or each ingredient can be separately prepared and then mixed stepwise.

For example, a process of preparing the bowel cleansing preparation of the present invention may comprise the steps of forming a first mixture comprising a sugar alcohol as a first cleansing ingredient; forming a second mixture comprising ascorbic acid or ascorbic acid and a salt of ascorbic acid as a second cleansing ingredient; as necessary, forming a third mixture comprising bisacodyl as a third cleansing ingredient; as necessary, forming a fourth mixture comprising an anti-foaming agent; as necessary, forming a fifth mixture comprising a gastrointestinal soother; and mixing the first mixture to fifth mixture with an aqueous solvent to form a bowel cleansing preparation.

In another aspect of the present invention, a process for preparing the bowel cleansing preparation of the present invention may additionally include the steps of mixing the first mixture and second mixture, and as necessary, the third mixture, fourth mixture, and fifth mixture in any combination prior to mixing the first mixture to fifth mixture with an aqueous solvent.

In a further aspect of the present invention, a process for preparing the bowel cleansing preparation of the present invention may involve, for example, preparing in advance a highly concentrated solution comprising the first mixture and second mixture, and as necessary, the third mixture, fourth mixture, and fifth mixture as well as part of an aqueous solvent and then adjusting the solution to the above-described specific concentrations and quantities by diluting with various beverages at the time of ingestion. In this case, the type of beverage that users can choose as a vehicle is not particularly limited as long as it is an aqueous solvent, including water, carbonated water, alkaline ionized water and a beverage, etc., and the beverage may be the same as or different from the aqueous solvent in the highly concentrated solution. There is no particular limitation on the kind of beverage as long as the effect of the present invention can be achieved. Examples of the beverage include drip coffee, various juices, cola, clear sodas, and gin and tonic, etc.

In the process for preparing the bowel cleansing preparation of the present invention, the method of mixing for the individual ingredients, particle shape and size of solids, pH, preparation temperature, agitation conditions, containers, packaging material, vacuum packaging or gas-substituted packaging, and other packaging specifics may be adjusted as necessary depending on the form, type, shipping and storage method of the bowel cleansing preparation to be prepared.

A dosing regimen for the bowel cleansing preparation of the present invention may involve, without limitation, consuming 50-100 cc of the composition every 5-10 minutes for a total of 5-10 doses, starting at 3-5 hours before the scheduled colonoscopy depending on the bowel sensitivity of the subject. While not intended to be limiting, the bowel cleansing preparation of the present invention may be ingested over a time period of, for example, 1 hour to 1.5 hour and a suitable amount of bottled water or the like may be additionally taken in case of thirst.

By comprising specific concentrations of a sugar alcohol and ascorbic acid and, as necessary, bisacodyl, etc., the bowel cleansing preparation of the present invention displays enhanced bowel cleansing efficacy despite a greatly reduced volume of ingestion. The bowel cleansing preparation of the present invention also achieves convenience of administration by requiring a very small volume of ingestion. In addition, the bowel cleansing preparation of the present invention causes no or minimal discomfort such as nausea, retching, and bloating as it does not contain unpalatable ingredients, and is safe as it inhibits the production of combustible gases in the colon.

The preparation process of the present invention allows for the economical mass production and commercialization of a high performance bowel cleansing preparation.

As the bowel cleansing preparation of the present invention has excellent cleansing efficacy, ease of administration and safety, it can be utilized for bowel preparation prior to colonoscopy or the like, for preparation prior to colonic surgery and anal surgery for hemorrhoid or the like as well as for the treatment and alleviation of diseases such as chronic and acute constipation.

The bowel cleansing preparation of the present invention may not only be used alone, but it may also be used in combination with existing bowel cleansing preparations, e.g., PICOLIGHT or PEG-based formulations such as COLYTE powder or CoolPrep. The combined use with existing products such as COLYTE powder, PICOLIGHT, CoolPrep, etc. may bring about the synergistic effect of enhancing the degree of bowel cleansing while reducing the volume of ingestion. That is, when the bowel cleansing preparation of the present invention is used in combination with existing bowel cleansing preparations, using as little as half its usual dose can reduce the volume of ingestion of the existing bowel cleansing preparations from 3-4 L to 1 L or less. The composition of the present invention can also be used as a solvent for or as a fluid to be mixed with existing bowel cleansing preparations, enhancing the efficacy of the existing bowel cleansing preparations and thereby allowing for the total amount of the main ingredient of the existing cleansing preparations such as PEG or picosulfate as well as the total required volume of the cleansing solution to be reduced to 30% or less.

The present invention will now be described in more detail with reference to examples. It is to be understood, however, that the examples are provided to illustrate embodiments of the present invention and not intended to limit the scope of the invention.

### <Example 1>

In 100 mL of carbonated water as solvent, 46 g of ascorbic acid, 2 g of calcium ascorbate, 40 g of sorbitol, 10 mg of bisacodyl, 1.5 g of citric acid, 33.5 mg of docusate sodium, 20 mg of caffeine, 100 mg of pectin, 30 mg of zinc oxide, 300 mg of simethicone, 1.5 g of sodium bicarbonate, 2 g of potassium bicarbonate, and 35 mg of sucralose were mixed to form 150 ml of a highly concentrated solution, and 350 ml of carbonated water was used as a separate vehicle to prepare a two-part bowel cleansing preparation.

Mixing was conducted by mixing all the ingredients in powder form at the same time and then pouring carbonated water to dissolve them. Caution is needed because a large amount of carbon dioxide (CO₂ gas generated by the neutralization reaction between the acidic ascorbic acid and citric acid and basic bicarbonate salt causes extensive foaming. Preparing a 1:10 diluted solution of Gasocol 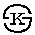 containing the anti-foaming agent simethicone beforehand and adding it suitably while mixing can successfully prevent foaming, thereby facilitating the mixing process.

Among the ingredients listed above, pectin needs vigorous stirring for its complete dissolution in the solvent because, being a dietary fiber, it is not easy to be dissolved. Because pharmaceutical grade pure simethicone containing about 30% of silicone is insoluble in water, Gasocol 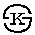 , which is a liquid formulation comprising simethicone as the main component, was used instead in this example.

### <Example 2>

In 100 mL of bottled water as solvent, 30 g of ascorbic acid, 60 g of xylitol, 10 mg of bisacodyl, 1.5 g of citric acid, 300 mg of simethicone, 5 g of potassium bicarbonate, and 35 mg of sucralose were mixed to form 150 ml of a highly concentrated solution, and 350 ml of bottled water was used as a separate vehicle to prepare a two-part bowel cleansing preparation. Mixing of the ingredients was conducted in the same manner as in Example 1.

### <Reference Examples 1 to 6>

For *in vitro* tests to assess the safety of sugar alcohols used as a first cleansing ingredient in the present invention, Reference Examples 1 to 6 were prepared for comparison with the solution of Example 1.

Reference Examples 1 to 6 refer to a COLYTE solution, a PICOLIGHT solution, a CoolPrep solution, a sorbitol (100 g/L) solution, a mannitol (100 g/L) solution, and a 12% xylitol (120 g/L) solution, respectively, with the COLYTE solution, PICOLIGHT solution, CoolPrep solution prepared by diluting the respective active ingredients in bottled water according to the manufacturer's instructions such that the concentration of each active ingredient is the same as its concentration used in the bowel preparation.

### <Experimental Example 1: Safety tests concerning the production of combustible gases (in vitro)>

### Concentrations of hydrogen and methane measurement

With regard to safety tests regarding the production of combustible gases in the colon, it is not easy to obtain suitable specimens from the subject or assess colonic gas during colonoscopy and it is not reasonable to conclude that the observed differences are attributable to the difference in the bowel cleansing solution based only on results obtained from a limited number of cases while the conditions of individual subjects after bowel preparation are widely different. Under the circumstances, *in vitro* tests, which can be conducted under identical conditions, were utilized to indirectly assess the potential risks of the composition prepared in Example 1.

For this purpose, aliquots of the solutions from Example 1, Reference Examples 1 to 5, and control (bottled water) were mixed with a diluted solution of stool collected from 5 people and stored in 500 ml containers for 18 hours at room temperature.
Then, gas concentrations in each container were measured using a gas detector. The diluted solutions of stool were prepared by sampling stools from five different subjects and diluting 3 g of the stool from each subject in 100 ml of bottled water, giving a total of 5 different diluted solution of stool.

Detailed test procedures are described below.

For each of the five subjects whose stool samples were taken, a 10 ml aliquot of the diluted solution of stool was placed in each of the seven 500 ml containers, providing seven containers holding the same diluted solution of stool. As a result, a total of 35 containers holding the diluted solutions of stool derived from the five subjects were prepared.

For each subject, 10 ml each of the test solutions from Example 1, Reference Examples 1 to 5 (COLYTE, PICOLIGHT, CoolPrep, sorbitol, mannitol solution, respectively) and Control (bottled water) were added to the seven containers holding the same diluted solution of stool, respectively.

After mixing the diluted solutions of stool with the respective test solutions, the containers were sealed by tight capping and stored for 18 hours at room temperature.

Then, the concentrations of hydrogen and methane in each container were measured using the Geotech GA5000 gas analyzer (Landtech, UK). Geotech GA5000 gas analyzer can measure methane in vol % unit and hydrogen in the range of 0-1000 ppm.

Measurement of the gas concentrations in the 35 containers in total gave average measurements for the concentrations of the combustible gases, hydrogen and methane, as shown in Table 1.

**[Table 1]**

| | Example 1 | Reference Example 1 (COLYTE) | Reference Example 2 (PICOLIGHT) | Reference Example 3 (CoolPrep) | Reference Example 4 (Sorbitol) | Reference Example 5 (Mannitol) | Control (Bottled water) |
|---|---|---|---|---|---|---|---|
| Hydrogen (ppm) | 0 | 0 | 51 | 0 | 715 | 757.8 | 173.6 |
| Methane (Vol%) | 0.14 | 0.16 | 0.16 | 0.16 | 0.16 | 0.18 | 0.14 |

As can be seen from Table 1, although it comprises sorbitol at a concentration of 80 g/L, the bowel cleansing preparation of Example 1 gave no detectable hydrogen, as well as a methane concentration of 0.14%, which is equal to that for the control and lower than those for the bowel cleansing solutions of Reference Examples 1 to 5. These results show that the bowel cleansing preparation according to the present invention was the safest with respect to the generation of colonic combustible gases.

### Bacterial culture test

### Following the measurement of gas concentration as described above, the mixed solutions in the containers were submitted to a laboratory at the Seoul Medical Science Institute (SCL), a CAP accredited and Korean Society for Laboratory Medicine (KSLM)/Laboratory Medicine Foundation accredited institution, where they were cultured for 48 hours and measured for the resulting colony counts.

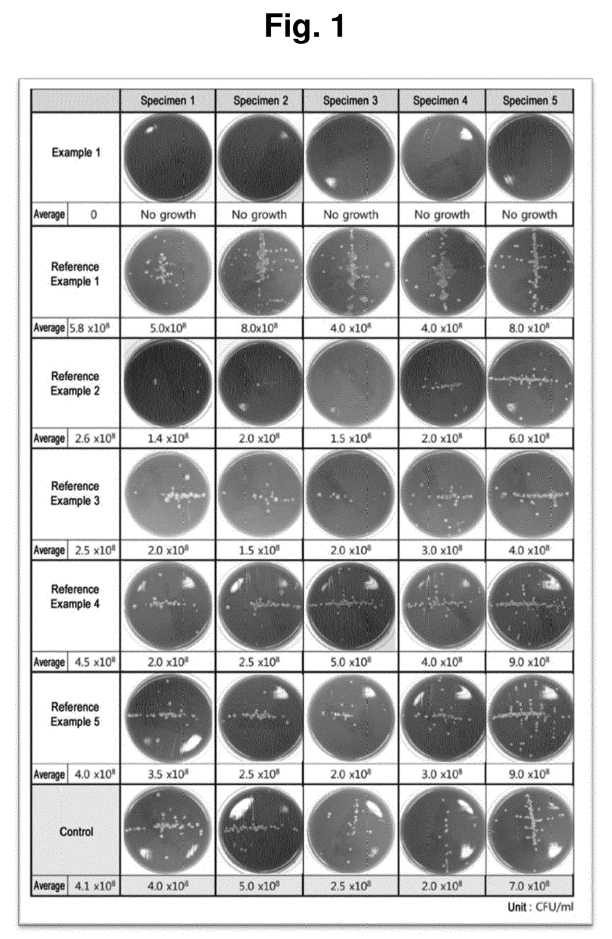

Averages of the colony counts for the respective test solutions are listed in Table 2. In addition, Fig. 1 shows the results from the culture of intestinal bacteria for Example 1, Reference Examples 1 to 5, and Control.

**[Table 2]**

| | Example 1 | Reference Example 1 (COLYTE) | Reference Example 2 (PICOLIGHT) | Reference Example 3 (CoolPrep) | Reference Example 4 (Sorbitol) | Reference Example 5 (Mannitol) | Control (Bottled water) |
|---|---|---|---|---|---|---|---|
| Colony count (CFU/mL) | 0 | 5.8x10⁸ | 2.5x10⁸ | 2.6x10⁸ | 4.5x10⁸ | 4.0x10⁸ | 4.1x10⁸ |

The above colony count results clearly demonstrate the antibacterial effect of the bowel cleansing preparation of the present invention. Specifically, for Example 1, bacterial growth was not observed in any of the cultures derived from the diluted solutions of stool collected from 5 subjects. It can be seen that for Example 1, bacteria capable of producing combustible gases such as hydrogen or methane were completely killed. Although obtained from *in vitro* tests, these results demonstrate that with respect to the production of combustible gases in the colon, the bowel cleansing preparation of the present invention is safer than any of the existing bowel cleansing preparations.

With respect to Example 2 in which xylitol was used as the first cleansing ingredient, in order to test the antibacterial effect of the solution against intestinal bacteria, a 5% diluted solution of stool was prepared from a stool sample obtained from one subject. A 10 ml aliquot of the stool suspension was placed in each of 6 containers numbered 1 to 6, to which 15 ml each of the Example 2 solution, COLYTE solution (Reference Example 1), PICOLIGHT solution (Reference Example 2), CoolPrep solution (Reference Example 3), 12% xylitol solution (Reference Example 6), and bottled water (Control) were added, respectively. Then, each of these mixed solutions was split in half and submitted to two different testing institutions, the Seoul Medical Science Institute (SCL), which is a CAP accredited and KSLM/Laboratory Medicine Foundation accredited institution, and the Green Cross Laboratories (GC Labs), which is a KSLM accredited institution, for bacterial culture tests.

The results of the bacterial culture tests are shown in Table 3.

**[Table 3]**

| | Example 2 | Reference Example 1 (COLYTE) | Reference Example 2 (PICOLIGHT) | Reference Example 3 (CoolPrep ) | Reference Example 6 (Xylitol) | Control (Bottled water) |
|---|---|---|---|---|---|---|
| Colony count (CFU/mL) (GCLabs) | 2.2×10⁶ | 14.8×10⁶ | 17.3×10⁶ | 7.3x10⁶ | 10.2×10⁶ | 11.6×10⁶ |
| Colony count (CFU/mL) (SCL) | 0.9×10⁶ | 150×10⁶ | 150×10⁶ | 60×10⁶ | 60×10⁶ | 60x 10⁶ |

The results in Table 2 show that the colony count was lowest in the culture of the diluted solution of stool to which the solution of Example 2 was added. In addition, it can be seen that the diluted solution of stool to which a 12% xylitol solution (the same xylitol concentration as in Example 2) was added also gave a colony count similar to that of the control. These results indirectly confirm the antibacterial effect of xylitol, indicating that xylitol will not promote the growth of intestinal bacteria when used as the main ingredient of a bowel cleansing solution.

The above results demonstrate that the preparation of Example 2 comprising xylitol and ascorbic acid as main ingredients is very safe with regard to intestinal bacterial growth and the resulting production of combustible gases in the colon.

Such excellent antibacterial effect may greatly contribute to reducing the incidence of postoperative infections when the bowel cleansing preparation of the present invention is used as a laxative for bowel preparation before colonic surgery. Because contamination from the bowel contents may occur during colonic surgery, the preoperative administration of antibiotics to prevent surgical site infection due to such contamination is established as the standard. However, if the bowel cleansing preparation of the present invention is used for preoperative bowel preparation, it kills or inhibits bacteria in the bowel contents, significantly reducing the possibility of infection even when contamination from the bowel contents occurs.

In view of the fact that existing bowel cleansing preparations are used not only for bowel preparation prior to colonoscopy but also as the main laxative for preoperative bowel preparation before colonic surgery, the above-described effect may enhance the significance of the bowel cleansing preparation of the present invention.

From the tw*o in vitro* safety tests described above, it can be seen that the novel bowel cleansing preparation according to the present invention has the lowest risk as it minimizes the production of combustible hydrogen and methane gases compared to conventional colon cleansing preparations and inhibits bacterial growth.

### <Comparative Examples 1 to 3>

Comparative Examples 1 to 3 were prepared for comparative experiments concerning the ease of administration, bowel cleansing efficacy and safety of the bowel cleansing preparation of the present invention.

Specifically, *for in vivo* experiments, commercially available bowel cleansing products COLYTE, PICOLIGHT, and CoolPrep were respectively mixed with water in their recommended mixing amounts to prepare the bowel cleansing preparations for Comparative Examples 1 to 3. The identities of the bowel cleansing agents and the volume of water used in Comparative Examples 1 to 3 are listed in Table 4.

**[Table 4]**

| | Bowel cleansing agent | Volume of water |
|---|---|---|
| Comparative Example 1 | COLYTE 8 pouches | 4L |
| Comparative Example 2 | PICOLIGHT 3 pouches | 3.45 L |
| Comparative Example 3 | CoolPrep 4 pouches | 3 L (including 1 L water for additional ingestion) |

| | | |
|---|---|---|
| - COLYTE: Taejoon Pharm Co., Ltd., 34.65 g per pouch - PICOLIGHT: Pharmbio Korea Co., Ltd., 16.37 g per pouch - CoolPrep: Taejoon Pharm Co., Ltd., 56.402 g per pouch | | |

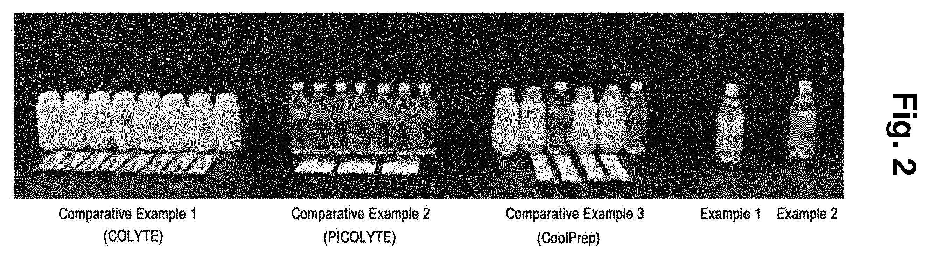

Fig. 2 is a photograph of the bowel cleansing preparations of Comparative Examples 1 to 3 before mixing the cleansing ingredients with the solvent and those of Examples 1 and 2 after mixing the cleansing ingredients with the solvent, which shows the dosage for the preparations of Example 1 and Comparative Examples 1 to 3. As shown in Fig. 2, Examples 1 and 2 have dosages that are drastically lowered to a level of 1/8 to 1/6 compared to Comparative Examples 1 to 3.

The bowel cleansing preparations prepared in Example 1, Example 2 and Comparative Examples 1 to 3 were evaluated for their cleansing performance and compliance.

Specifically, the bowel cleansing efficacy, ease of administration, and safety of Comparative Example 1 (n=8), Comparative Example 2 (n=45), Comparative Example 3 (n=49), Example 1 (n=48) and Example 2 (n=50) were evaluated in patients undergoing colonoscopy. The reason for the small subject population in Comparative Example 1 is that there was a limit in recruiting subjects as patients actively avoided the COLYTE powder due to the widespread perception of discomfort associated with ingesting 4 L of the preparation.

Detailed dosing regimens for Examples 1 and 2 and Comparative Examples 1 to 3 are as follows.

In the dosing regimen for Examples 1 and 2, starting from 5 hours before the scheduled examination, a total of 500 ml of the bowel cleansing solution was ingested at a rate of 100 ml (5 mouthfuls, one half of a paper cup) every 10 minutes for a total of 5 times as a general rule. According to this regimen, ingestion of the bowel cleansing agent took 40 to 50 minutes in total.

With respect to Comparative Example 1, i.e., COLYTE powder 4 L, the recommended dosing regimen in the product manual was followed. Starting from 6 hours before the scheduled examination, 250 ml of the cleansing preparation was taken every 10 minutes for a total of 16 times, which took 2 hours and 30 minutes in total.

With respect to Comparative Example 2, i.e., PICOLIGHT 3.45 L, one pouch provided in the product was ingested after dilution in 150 ml of water at 7 PM the day before the examination followed by additional 1 L of water over an hour, and the process was repeated twice on the day of examination at 5 hours and 4 hours before starting the examination.

With respect to Comparative Example 3, i.e., CoolPrep 3 L, 1 L of the prepared solution was ingested over 1 hour (250 ml of solution every 15 minutes) at 7 PM the day before the examination, and another 1 L of the prepared solution was ingested over 1 hour on the day of examination at 5 hours before starting the examination. The patients were instructed to additionally drink 500 ml of water in the evening and morning after completing the ingestion of the diluted solution.

### <Experimental Example 2: Bowel cleansing efficacy>

Bowel cleansing efficacy was assessed in two aspects: ① colon cleanliness and ② the amount of bubbles.

### Colon cleanliness

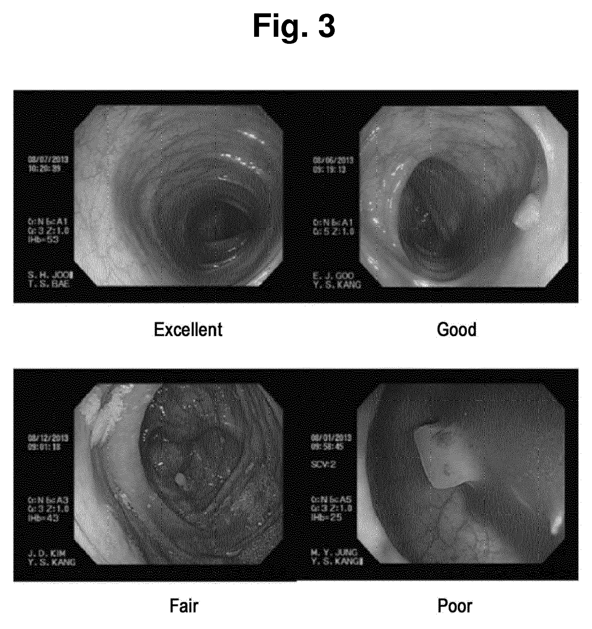

For the assessment of colon cleanliness, a surgeon evaluated the level of colon cleanliness of a patient using a five-point scale (Excellent, Good, Fair, Poor, Fail) according to the criteria shown in Table 5 and Fig. 3. For a fair evaluation, the evaluation was carried out in a blind test in which the surgeon performing the colonoscopy was not informed of the identity of the bowel cleansing preparation consumed by the individual patients.

**[Table 5]**

| | |
|---|---|
| Excellent | Very thorough cleansing allowing the detection of even small lesions |
| Good | Slightly less clean than "Excellent" while sufficiently clean to allow the detection of even small lesions |
| Fair | Possible to miss one or two lesions ≤ 5mm |
| Poor | Possible to miss lesions ≥ 5mm due to poor cleanliness but no possibility of missing malignant lesions, i.e., colon cancer |
| Fail | Requiring repeat preparation because of the possibility of missing malignant lesions, i.e., colon cancer |

The results of the colon cleanliness assessment according to the criteria shown in Table 5 and Fig. 3 are provided in Table 6.

**[Table 6]**

| Sample No. | Colon cleanliness (%) | | | | |
|---|---|---|---|---|---|
| | Excellent | Good | Fair | Poor | Fail |
| Example 1 | 50 | 41.7 | 4.2 | 2.1 | 0 |
| Example 2 | 60 | 34 | 6 | 0 | 0 |
| Comparative Example 1 (COLYTE) | 12.5 | 62.5 | 25 | 0 | 0 |
| Comparative Example 2 (PICOLIGHT) | 17.8 | 33.3 | 31.1 | 6.7 | 11.1 |
| Comparative Example 3 (CoolPrep) | 16.3 | 51 | 22.5 | 10.2 | 0 |

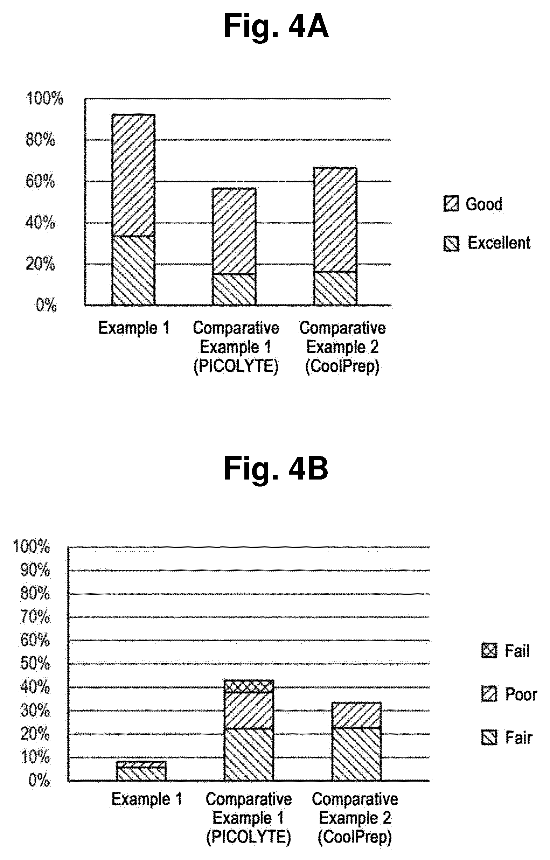

Fig. 4a and Fig. 4b present the above results classified as "effective bowel preparation" referring to conditions adequate for colonoscopy and "insufficient bowel preparation" referring to conditions unsatisfactory or impossible for accurate colonoscopy. "Effective bowel preparation" includes conditions rated as Excellent or Good, and "insufficient bowel preparation" includes conditions rated as Fair, Poor, or Fail.

As can be seen from Table 6, Fig. 4a and Fig. 4b, the rate of effective bowel preparation was the highest in Example 2 as 94% followed by 91.7% in Example 1 while Comparative Example 2 shown to be the worst in cleanliness with a rate of effective bowel preparation as low as 50%. Comparative Example 1 and Comparative Example 3 also showed rates of effective bowel preparation of only 75% and 67.3%, respectively. These results fairly correspond with reports in some foreign research articles indicating that the PEG preparations and phosphate preparations show rates of adequate bowel preparation of about 70∼75% <"Colonoscopy preparation", ASGE technology committee, GASTROINTESTINAL ENDOSCOPY, 69(No.7):1201-1209, 2009>.

However, insufficient bowel preparation as is seen with Comparative Examples 1 to 3 actually causes serious problems because it does not allow for accurate colonoscopy. Normally, colonoscopy is repeated every 4-5 years. If the results of colonoscopy performed after a painful bowel preparation are inaccurate such that polyps or early colon cancer are not detected, the diseases are usually neglected for 4-5 years until the next screening examination, potentially allowing for polyps to advance to cancer or for the early colon cancer to advanced colon cancer.

### Amount of bubbles

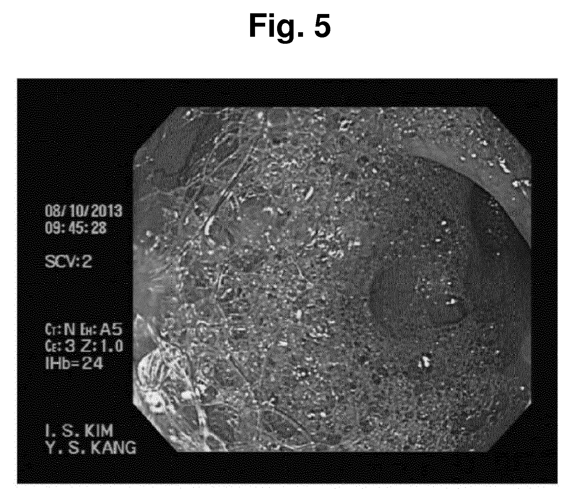

In addition to colon cleanliness, another major factor for accurate colonoscopy is the presence of bubbles in the colon. Almost all bowel cleansing agents tend to generate bubbles in the colon following bowel preparation. Accordingly, the prevention or removal of such bubbles is required in order to thoroughly inspect the colonic lumen to detect even small lesions. The presence or absence as well as the extent of bubbles were observed after ingestion of the bowel cleansing preparations of Examples 1 and 2 and Comparative Examples 1 to 3, and the results are shown in Table 7. The extent of bubbles were rated on a three-point scale of "None," "Some" and "Substantial." Fig. 5 is a photograph showing a substantial amount of intracolonic bubbles generated after the consumption of the bowel cleansing preparation of a Comparative Example.

**[Table 7]**

| Samples | Amount of bubbles (%) | | |
|---|---|---|---|
| | None | Some | Substantial |
| Example 1 | 85.4 | 14.6 | 0 |
| Example 2 | 88 | 12 | 0 |
| Comparative Example 1 (COLYTE) | 28.6 | 42.9 | 14.3 |
| Comparative Example 2 (PICOLIGHT) | 31.6 | 34.2 | 34.2 |
| Comparative Example 3 (CoolPrep) | 32.5 | 27.5 | 40 |

As shown in the above results, with Examples 1 and 2, bubbles were not generated in 85.4% and 88% of the subjects, respectively. However, with Comparative Examples 1 to 3, the percentage of subjects developing no bubbles was as low as about 30%. In particular, with Comparative Examples 2 and 3, substantial amounts of bubbles were formed to the extent that examination was greatly interfered in more than one in three subjects. When bubbles form, they can be washed away using pharmaceutical agents during colonoscopy. However, it takes a substantial amount of effort and time as the area that can be be washed at one time is limited. Thus, excessive bubble formation interferes with the smooth progress of colonoscopy and also becomes the reason for missing small lesions. As for the bowel cleansing preparations of Examples 1 and 2, the almost complete prevention of bubble formation allows for more time to be allotted to the observation of lesions while at the same time reducing procedure time, and also enhances the disease detection rate because the clear vision facilitates the detection of small lesions.

### <Experimental Example 3: Ease of administration >

Ease of administration was assessed in four aspects: ① taste of the cleansing agent, ② discomfort after administration, ③ willingness to recommend to family, and ④ need for improvement.

### Taste of cleansing agents

In a questionnaire, the subjects were asked to subjectively rate the taste of the bowel cleansing agents on a three-point scale with "Very unpleasant", "Slightly unpleasant", and "Fair." The percentages of each response category are shown in Table 8.

**[Table 8]**

| Sample | Taste of bowel cleansing preparations (%) | | |
|---|---|---|---|
| | Very unpleasant | Slightly unpleasant | Fair |
| Example 1 | 2.1 | 12.5 | 85.4 |
| Example 2 | 0 | 10 | 90 |
| Comparative Example 1 (COLYTE) | 12.5 | 37.5 | 50 |
| Comparative Example 2 (PICOLIGHT) | 2.2 | 4.4 | 93.4 |
| Comparative Example 3 (CoolPrep) | 14.3 | 53.1 | 32.6 |

As shown in the above results, with Examples 1 and 2, 85.4% and 90% of the subjects, respectively, rated the taste of the bowel cleansing agents as "Fair," which are slightly lower than that with Comparative Example 2. With Comparative Example 1 and Comparative Example 3, however, only 50% and 32.6% of the subjects, respectively, rated the taste of the bowel cleansing agents as "Fair." In particular, with Comparative Example 3, the percentage of subjects who found the taste of the bowel cleansing agent unpleasant was higher than that with Comparative Example 1 despite the fact that the volume of ingestion was reduced to 3/4 compared with Comparative Example 1.

### Discomfort after administration

The discomforts after administration were classified into 4 groups of abdominal pain, bloating, vomiting, and thirst. In a questionnaire, the subjects were asked to indicate self-perceived discomforts, and the percentages of each response category are shown in Table 9.

**[Table 9]**

| Sample | Discomforts after administration (%) | | | |
|---|---|---|---|---|
| | Abdominal pain | Bloating | Vomiting | Thirst |
| Example 1 | 10.4 | 12.5 | 6.3 | 21.2 |
| Example 2 | 10 | 12 | 8 | 18 |
| Comparative Example 1 (COLYTE) | 0 | 12.5 | 37.5 | 0 |
| Comparative Example 2 (PICOLIGHT) | 20.9 | 18.6 | 9.3 | 14 |
| Comparative Example 3 (CoolPrep) | 10.4 | 10.4 | 37.5 | 18.8 |

As can be seen from Table 9, Examples 1 and 2 caused a level of discomfort that is lower than or similar to existing bowel cleansing agents with regard to the items of abdominal pain, bloating and vomiting. Although Example 1 caused a relatively high incidence of thirst, this seems to be attributable to the relatively small volume of water ingested. Additional water intake by the subjects was restricted during the tests in order to accurately evaluate the effects of the bowel cleansing agents, which may also have affected the above results. In fact, the rate of subjects complaining of thirst reduced significantly when, after the test was completed, the subjects were allowed to additionally drink 1-2 cups of water as necessary. In comparison, vomiting was quite frequent in Comparative Example 1 and Comparative Example 3, which appears to be associated with the unpleasant taste of PEG preparations.

### Willingness to recommend

In a questionnaire, subjects were asked if they are willing to recommend the bowel cleansing preparation they used to their family, and the percentages of subjects who answered negatively are shown in Table 10.

**[Table 10]**

| Sample | Non-recommendation (%) |
|---|---|
| Example 1 | 6.3 |
| Example 2 | 0 |
| Comparative Example 1 (COLYTE) | 62.5 |
| Comparative Example 2 (PICOLIGHT) | 8.9 |
| Comparative Example 3 (CoolPrep) | 53 |

As shown in the above results, the percentage of non-recommendation was highest in Comparative Example 1 where the preparation has an unpleasant taste and a large volume of ingestion, with 62.5% of the subjects replying that they are not willing to recommend the preparation. Comparative Example 3 also showed a substantially high percentage of non-recommendation, 53%. The fact that Comparative Example 3 (CoolPrep) with a 3 L volume of ingestion showed a percentage of non-recommendation that is 6 times higher than that of Comparative Example 2 (PICOLIGHT) with 3.45 L volume of ingestion (8.9% vs. 53%) appears to highlight the non-preference for PEG preparations.

In the case of Comparative Example 2 which showed the lowest cleansing efficacy, while the percentage of "Fair" in the taste of the cleansing agent was 93.4%, higher than Example 1 (85.4%) and Example 2 (90%), the percentage of "Non-recommendation" was also slightly higher than Examples 1 and 2. This may be associated with its large volume of ingestion amounting to 3.45 L.

### Need for improvement

In a questionnaire, the subjects were asked if the bowel cleansing preparation they used needs improvement, and the percentages of subjects who answered affirmatively are shown in Table 11.

**[Table 11]**

| Sample | Need for improvement (%) |
|---|---|
| Example 1 | 8.3 |
| Example 2 | 8 |
| Comparative Example 1 (COLYTE) | 62.5 |
| Comparative Example 2 (PICOLIGHT) | 35 |
| Comparative Example 3 (CoolPrep) | 58 |

From the above results, it can be seen that Example 2 shows the highest satisfaction as the percentage of subjects who saw the need for improvement is the lowest. In comparison, the fact that 35% of the subjects saw the need for improvement in Comparative Example 2, i.e., PICOLIGHT, which showed high compliance in terms of the taste and willingness to recommend, appears to be a meaningful inconsistency when compared to the percentage of "Non-recommendation", 8.9%. This can solely be interpreted as a demand for improvement in the excessively large volume of ingestion amounting to 3.45 L. In light of the above, while the taste of a bowel cleansing agent is a factor with great impact on the ease of administration, the volume of ingestion is also a significant factor for the patients' satisfaction.

### Patient preferences for Example 1 and other bowel cleansing agents

Among the subjects who were given the bowel cleansing preparation of Example 1, 23 subjects who had previously experienced colon preparation were asked if the ease of administration was enhanced compared to their previous colon preparation experiences. The results are shown in Table 12.

**[Table 12]**

| Relative Ease of Use | | Percentage of subjects having experience with other bowel cleansing agents (%) |
|---|---|---|
| Easier | Much easier | 51.8 |
| | Slightly easier | 31 |
| Comparable | | 17.2 |
| More difficult | | 0 |

As can be seen from the above results, 83% of the subjects who had experienced other bowel cleansing agents replied that consumption of the bowel cleansing preparation of Example 1 was easier, while none of the subjects found it more difficult. These results allows for an indirect comparison between the existing bowel cleansing agents in Comparative Examples 1 to 3 and the preparation of Example 1, which shows a significantly favorable preference for the preparation of Example 1.

### <Experimental Example 4: Safety (in vivo test)>

The safety of each bowel cleansing agent was assessed by conducting blood tests for the blood ascorbic acid concentration, and blood chemistry.

### Colonic gas measurement

With respect to the production of combustible gases, further to the above *in vitio* test showing the safety of the bowel cleansing preparation of the present invention, for each of the 10 subjects who used the bowel cleansing preparation of Example 1 or 2, actual colonic gas collected during their colonoscopy was analyzed.

The results showed that the methane concentration was 0.1∼0.2 vol% and the hydrogen concentration was 1∼4 ppm, directly demonstrating that the concentrations of these gases are in no way close to their minimum explosive concentrations, i.e., 5% and 4,000 ppm, respectively.

### ]Blood ascorbic acid concentration

Following the consumption of the bowel cleansing preparations, blood samples were taken from subjects just before their colonoscopy, and the blood ascorbic acid concentrations were measured at the SCL (Seoul Medical Science Institute), a testing institution equipped with apparatus for measuring ascorbic acid blood levels. The resulting average values are listed in Table 13.

**[Table 13]**

| Sample | Average blood ascorbic acid concentration (Reference range: 2-20 μg /mL) |
|---|---|
| Example 1 | 37.4 |
| Example 2 | 33.7 |
| Comparative Example 1 | 7.6 |
| Comparative Example 2 | 6.9 |
| Comparative Example 3 | 30.4 |

The reference range for blood ascorbic acid concentration is 2∼20 μg/mL. It can be expected that the blood ascorbic acid concentration is transiently elevated above the reference range with Comparative Example 3 where 21.2 g of ascorbic acid is mixed in the ingested liquid as well as with Example 1 containing 48 g of ascorbic acid compounds and Example 2 containing 30 g of ascorbic acid. However, since ascorbic acid is water soluble, blood ascorbic acid exceeding the reference value is directly excreted via the kidney to reduce the blood concentration to normal.

In addition, the U.S. National Cancer Institute has officially confirmed that high dose ascorbic acid is not harmful to the human body (http://www.cancer.gov/cancertopics/pdq/cam/highdosevitaminc/healthprofessional/pag el/AllPages).

Since the 1970s, many studies have been conducted to determine if an intravenous injection of high dose ascorbic acid has therapeutic effects on various cancers. While there is no consensus on its therapeutic effect, it was confirmed along the way that except for people with G6PD deficiency, renal disorders or urolithiasis, up to 1.5 g/kg of ascorbic acid can be safely administered intravenously to a healthy person <"Vitamin C pharmacokinetics: implications for oral and intravenous use", Padayatty SJ, Sun H, Wang Y, et al., Ann Intern Med 140 (7), 533-7, 2004; "Phase I clinical trial of i.v. ascorbic acid in advanced malignancy", Hoffer LJ, Levine M, Assouline S, et al., Ann Oncol 19 (11), 1969-74, 2008>.

It was also reported in a phase I clinical study conducted in 2013 that ascorbic acid 15 g given intravenously over 30 minutes twice weekly for four weeks elevated the blood ascorbic acid level to at least 350 mg/dL (3,500 μg/mL), yet was very well tolerated and caused no severe adverse effects <"Pharmacological ascorbate with gemcitabine for the control of metastatic and node-positive pancreatic cancer (PACMAN): results from a phase I clinical trial", Welsh JL, Wagner BA, van't Erve TJ, et al., Cancer Chemother Pharmacol 71 (3), 765-75, 2013>.

The above information is found in official resources published on the website of the U.S. National Cancer Institute, confirming that high blood ascorbic acid concentration causes no harm to human health. In addition, the above-referenced ascorbic acid blood concentration of 3,500 μg/mL is almost 175 times the upper limit of its reference range and 94 times as high as 37.4 μg/mL, the average blood ascorbic acid concentration observed after using the bowel cleansing preparation of Example 1. Thus, it can be seen that the elevation of ascorbic acid concentration after consuming the bowel cleansing preparation of Example 1 or 2 does no harm to human health.

It is believed that for these reasons, MoviPrep^{®} was able to be approved by the U.S. FDA as a bowel cleansing agent without difficulty despite having the same composition as CoolPrep (used in Comparative Example 3), which has been shown to cause transient elevation of the blood ascorbic acid level.

### Other blood chemistry test

As other blood chemistry tests, the concentrations of essential trace elements, electrolyte concentrations, concentrations of blood urea nitrogen (BUN) and creatinine, which are indicators of kidney function, as well as liver enzymes AST(GOT) and ALT(GPT) reflecting liver damage were measured and the results are shown in Tables 14 to 17. Blood was collected right before the colonoscopy and measurements were taken at the Department of Laboratory Medicine at Joyful Hospital following the usual protocols.

**[Table 14]**

| Sample | P (Reference range: 2.5-5.5 mg/dL) | Mg (Reference range: 1.58-2.55 mg/dL) | Ca (Reference range: 8.6-10.2 mg/dL) |
|---|---|---|---|
| Example 1 | 4.6 | 2.4 | 9.9 |
| Example 2 | 4.5 | 2.3 | 9.7 |
| Comparative Example 1 (COLYTE) | 3.6 | 2.2 | 9.6 |
| Comparative Example 2 (PICOLIGHT) | 3.8 | 2.6 | 9.6 |
| Comparative Example 3 (CoolPrep) | 3.9 | 2.2 | 9.8 |

**[Table 15]**

| Sample | Na (Reference range: 135-145 mmol/L) | K (Reference range: 3.5-5.5 mmol/L) | Cl (Reference range: 98-110 mmol/L) |
|---|---|---|---|
| Example 1 | 144.5 | 4.5 | 107.2 |
| Example 2 | 141 | 4.8 | 105 |
| Comparative Example 1 (COLYTE) | 143.3 | 4.4 | 103.5 |
| Comparative Example 2 (PICOLIGHT) | 141 | 4.3 | 99.3 |
| Comparative Example 3 (CoolPrep) | 144.9 | 4.6 | 108.2 |

**[Table 16]**

| Sample | BUN (Reference range: 5-23 mg /dL) | Creatinine (Reference range: 0.5-1.2 mg /dL) |
|---|---|---|
| Example 1 | 12.8 | 0.9 |
| Example 2 | 9.3 | 0.9 |
| Comparative Example 1 (COLYTE) | 8.9 | 0.8 |
| Comparative Example 2 (PICOLIGHT) | 10.2 | 0.8 |
| Comparative Example 3 (CoolPrep) | 12.4 | 0.8 |

**[Table 17]**

| Samples | AST(GOT) (reference range: 0-32 IU/L) | ALT(GPT) (reference range: 0-31 IU/L) |
|---|---|---|
| Example 1 | 27.5 | 25.3 |
| Example 2 | 29.7 | 23.6 |
| Comparative Example 1 (COLYTE) | 25.9 | 19.7 |
| Comparative Example 2 (PICOLIGHT) | 27 | 26.6 |
| Comparative Example 3 (CoolPrep) | 28 | 24.3 |

As can be seen from the above results, except for the fact that the magnesium concentration was slightly elevated to 2.6 mg/dL (reference range 1.58-2.55 mg/dL) in subjects who took the bowel cleansing preparation of Comparative Example 2, no abnormalities were observed in the results for the concentrations of essential trace elements and electrolytes, kidney function indicators BUN and creatinine, and liver enzymes AST and ALT, for all of the bowel cleansing agents.

The reason the magnesium blood concentration was elevated beyond its reference range in patients who used the bowel cleansing preparation of Comparative Example 2 is that the ingredients of PICOLIGHT include a large amount of magnesium oxide (10.5 g).

To sum up, it has been shown that like other bowel cleansing agents, the bowel cleansing preparations of Examples 1 and 2 are safe formulations that do not cause hematochemical abnormalities.

## Claims

1. A bowel cleansing preparation for use in bowel cleansing comprising:
as a first cleansing ingredient, at least one sugar alcohol selected from xylitol, sorbitol, glycerol, erythritol, threitol, arabitol, ribitol, galactitol, fucitol, iditol, inositol, volemitol, isomalt, maltitol, lactitol, maltotriitol, maltotetraitol, and polyglycitol; as a second cleansing ingredient, ascorbic acid or a mixture of ascorbic acid and a salt of ascorbic acid; and an aqueous solvent,
wherein the concentration of said first cleansing ingredient is 10 g/L to 500 g/L based on the total preparation, the concentration of said second cleansing ingredient is 15 g/L to 500 g/L based on the total preparation, and the volume of said aqueous solvent is 0.1 L to 1.0 L.

2. The bowel cleansing preparation for the use of Claim 1, wherein the preparation further comprises, as a third cleansing ingredient, bisacodyl, and the concentration of said third cleansing ingredient is 5 mg/L to 50 mg/L based on the total preparation.

3. The bowel cleansing preparation for the use of Claim 2, wherein said third cleansing ingredient is incorporated in the form of a suspension.

4. The bowel cleansing preparation for the use of Claim 1, wherein the preparation further comprises an anti-foaming agent, and the concentration of said anti-foaming agent is 100 mg/L to 2 g/L based on the total preparation, and said anti-foaming agent is simethicone.

5. The bowel cleansing preparation for the use of Claim 1, wherein the preparation further comprises a cleansing ingredient selected from citric acid, magnesium, docusate sodium, senna extract, aloe extract, garlic extract, pectin, zinc, and caffeine.

6. The bowel cleansing preparation for the use of Claim 1, wherein the preparation further comprises a bicarbonate salt selected from sodium bicarbonate and potassium bicarbonate, and the concentration of said bicarbonate salt is 0.1 g/L to 10 g/L based on the total preparation in the case of sodium bicarbonate, and 0.1 g/L to 20 g/L based on the total preparation in the case of potassium bicarbonate.

7. The bowel cleansing preparation for the use of Claim 1, wherein the preparation further comprises a gastrointestinal soother selected from ginger, peppermint, and chamomile, and the concentration of said gastrointestinal soother is 5 g/L to 50 g/L based on the total preparation.

8. The bowel cleansing preparation for the use of Claim 1, wherein said first cleansing ingredient is xylitol.

9. The bowel cleansing preparation for the use of Claim 1, wherein said first cleansing ingredient is sorbitol.

10. The bowel cleansing preparation for the use of Claim 1, wherein said first cleansing ingredient is a mixture of xylitol and sorbitol.

11. The bowel cleansing preparation for the use of Claim 1, wherein, when said second cleansing ingredient is a mixture of ascorbic acid and a salt of ascorbic acid, the mass ratio of ascorbic acid to the salt of ascorbic acid ranges from 99:1 to 60:40.

12. The bowel cleansing preparation for the use of Claim 1, wherein said aqueous solvent is water, carbonated water, alkaline ionized water or a beverage.

13. The bowel cleansing preparation for the use of Claim 1, wherein said aqueous solvent is carbonated water.

14. The bowel cleansing preparation for the use of Claim 1, wherein the concentration of said first cleansing ingredient is 50 g/L to 150 g/L based on the total preparation.

15. The bowel cleansing preparation for the use of Claim 1, wherein the concentration of said second cleansing ingredient is 30 g/L to 200 g/L based on the total preparation.

16. The bowel cleansing preparation for the use of Claim 1, wherein the volume of said aqueous solvent is 0.3 L to 0.7 L.

17. The bowel cleansing preparation for the use of any one of Claims 1 to 16, wherein the preparation is a one-part product.

18. The bowel cleansing preparation for the use of any one of Claims 1 to 16, wherein the preparation is a two-part product.

19. The bowel cleansing preparation for the use of Claim 18, wherein the preparation is formulated into a two-part product consisting of (i) a package containing a highly concentrated solution formulated by mixing all ingredients in 0.05 L to 0.2 L of total volume of said aqueous solvent and (ii) a separate package containing the remaining amount of the aqueous solvent that is its total volume minus the volume used in formulating the highly concentrated solution.

## Patentansprüche

1. Darmreinigungszusammensetzung zur Verwendung in der Darmreinigung, umfassend: als ersten Reinigungsbestandteil, mindestens einen Zuckeralkohol, ausgewählt aus Xylitol, Sorbitol, Glycerol, Erythritol, Threitol, Arabitol, Ribitol, Galactitol, Fucitol, Iditol, Inositol, Volemitol, Isomalt, Maltitol, Lactitol, Maltotriitol, Maltotetraitol und Polyglycitol; als zweiten Reinigungsbestandteil, Ascorbinsäure oder ein Gemisch aus Ascorbinsäure und einem Salz von Ascorbinsäure; und ein wässriges Lösungsmittel,
wobei die Konzentration des ersten Reinigungsbestandteils 10 g/l bis 500 g/l, bezogen auf die gesamte Zusammensetzung, beträgt, die Konzentration des zweiten Reinigungsbestandteils 15 g/l bis 500 g/l, bezogen auf die gesamte Zusammensetzung, beträgt und das Volumen des wässrigen Lösungsmittels 0,1 1 bis 1,0 1 beträgt.

2. Darmreinigungszusammensetzung zur Verwendung nach Anspruch 1, wobei die Zusammensetzung weiterhin, als dritten Reinigungsbestandteil, Bisacodyl umfasst und die Konzentration des dritten Reinigungsbestandteils 5 mg/l bis 50 mg/l, bezogen auf die gesamte Zusammensetzung, beträgt.

3. Darmreinigungszusammensetzung zur Verwendung nach Anspruch 2, wobei der dritte Reinigungsbestandteil in Form einer Suspension eingebracht ist.

4. Darmreinigungszusammensetzung zur Verwendung nach Anspruch 1, wobei die Zusammensetzung weiterhin einen Entschäumer umfasst und die Konzentration des Entschäumers 100 mg/l bis 2 g/l, bezogen auf die gesamte Zusammensetzung, beträgt und der Entschäumer Simethicon ist.

5. Darmreinigungszusammensetzung zur Verwendung nach Anspruch 1, wobei die Zusammensetzung weiterhin einen Reinigungsbestandteil, ausgewählt aus Citronensäure, Magnesium, Docusat-Natrium, Senna-Extrakt, Aloe-Extrakt, Knoblauch-Extrakt, Pektin, Zink und Coffein, umfasst.

6. Darmreinigungszusammensetzung zur Verwendung nach Anspruch 1, wobei die Zusammensetzung weiterhin ein Hydrogencarbonatsalz, ausgewählt aus Natriumhydrogencarbonat und Kaliumhydrogencarbonat, umfasst und die Konzentration des Hydrogencarbonatsalzes 0,1 g/l bis 10 g/l, bezogen auf die gesamte Zusammensetzung, im Fall von Natriumhydrogencarbonat und 0,1 g/l bis 20 g/l, bezogen auf die gesamte Zusammensetzung, im Fall von Kaliumhydrogencarbonat beträgt.

7. Darmreinigungszusammensetzung zur Verwendung nach Anspruch 1, wobei die Zusammensetzung weiterhin ein Magen-Darm-Beruhigungsmittel, ausgewählt aus Ingwer, Pfefferminze und Kamille, umfasst und die Konzentration des Magen-Darm-Beruhigungsmittels 5 g/l bis 50 g/l, bezogen auf die gesamte Zusammensetzung, beträgt.

8. Darmreinigungszusammensetzung zur Verwendung nach Anspruch 1, wobei der erste Reinigungsbestandteil Xylitol ist.

9. Darmreinigungszusammensetzung zur Verwendung nach Anspruch 1, wobei der erste Reinigungsbestandteil Sorbitol ist.

10. Darmreinigungszusammensetzung zur Verwendung nach Anspruch 1, wobei der erste Reinigungsbestandteil ein Gemisch aus Xylitol und Sorbitol ist.

11. Darmreinigungszusammensetzung zur Verwendung nach Anspruch 1, wobei, wenn der zweite Reinigungsbestandteil ein Gemisch aus Ascorbinsäure und einem Salz von Ascorbinsäure ist, das Massenverhältnis von Ascorbinsäure zu dem Salz von Ascorbinsäure von 99:1 bis 60:40 reicht.

12. Darmreinigungszusammensetzung zur Verwendung nach Anspruch 1, wobei das wässrige Lösungsmittel Wasser, kohlensäurehaltiges Wasser, basisches ionisiertes Wasser oder ein Getränk ist.

13. Darmreinigungszusammensetzung zur Verwendung nach Anspruch 1, wobei das wässrige Lösungsmittel kohlensäurehaltiges Wasser ist.

14. Darmreinigungszusammensetzung zur Verwendung nach Anspruch 1, wobei die Konzentration des ersten Reinigungsbestandteils 50 g/l bis 150 g/l, bezogen auf die gesamte Zusammensetzung, beträgt.

15. Darmreinigungszusammensetzung zur Verwendung nach Anspruch 1, wobei die Konzentration des zweiten Reinigungsbestandteils 30 g/l bis 200 g/l, bezogen auf die gesamte Zusammensetzung, beträgt.

16. Darmreinigungszusammensetzung zur Verwendung nach Anspruch 1, wobei das Volumen des wässrigen Lösungsmittels 0,3 1 bis 0,7 1 beträgt.

17. Darmreinigungszusammensetzung zur Verwendung nach einem der Ansprüche 1 bis 16, wobei die Zusammensetzung ein einteiliges Produkt ist.

18. Darmreinigungszusammensetzung zur Verwendung nach einem der Ansprüche 1 bis 16, wobei die Zusammensetzung ein zweiteiliges Produkt ist.

19. Darmreinigungszusammensetzung zur Verwendung nach Anspruch 18, wobei die Zusammensetzung formuliert ist zu einem zweiteiligen Produkt, bestehend aus (i) einer Packung, die eine hochkonzentrierte Lösung, zubereitet durch Mischen aller Bestandteile in 0,05 1 bis 0,2 1 vom Gesamtvolumen des wässrigen Lösungsmittels, enthält, und (ii) einer separaten Packung, die die restliche Menge des wässrigen Lösungsmittels enthält, die dem Gesamtvolumen abzüglich des bei der Zubereitung der hochkonzentrierten Lösung verwendeten Volumens entspricht.

## Revendications

1. Composition de lavage intestinal, destinée au lavage colique, comprenant :
en tant que premier ingrédient de lavage, au moins un alcool de sucre sélectionné entre xylitol, sorbitol, glycérol, érythritol, thréitol, arabitol, ribitol, galactitol, fucitol, iditol, inositol, volémitol, isomalt, maltitol, lactitol, maltotriitol, maltotétraitol, et polyglycitol ; en tant que deuxième ingrédient de lavage, acide ascorbique ou un mélange d'acide ascorbique et d'un sel d'acide ascorbique ; et un solvant aqueux,
où la concentration du premier ingrédient de lavage est comprise entre 10 g/l et 500 g/l de la composition totale, la concentration du deuxième ingrédient de lavage est comprise entre 15 g/l et 500 g/l de la composition totale, et le volume du solvant aqueux est compris entre 0,1 1 et 1,0 l.

2. Composition de lavage intestinal destinée à l'usage selon la revendication 1, où la composition comprend en outre du bisacodyl en tant que troisième ingrédient de lavage, et où la concentration dudit troisième ingrédient de lavage est comprise entre 5 mg/l et 50 mg/l de la composition totale.

3. Composition de lavage intestinal destinée à l'usage selon la revendication 2, où le troisième ingrédient de lavage est incorporé sous la forme d'une suspension.

4. Composition de lavage intestinal destinée à l'usage selon la revendication 1, où la composition comprend en outre un agent anti-moussant, et où la concentration dudit agent anti-moussant est comprise entre 100 mg/l et 2 g/l de la composition totale, et où ledit agent anti-moussant est la siméthicone.

5. Composition de lavage intestinal destinée à l'usage selon la revendication 1, où la composition comprend en outre un ingrédient de lavage sélectionné entre acide citrique, magnésium, docusate de sodium, extrait de séné, extrait d'aloès, extrait d'ail, pectine, zinc et caféine.

6. Composition de lavage intestinal destinée à l'usage selon la revendication 1, où la composition comprend en outre un sel de bicarbonate sélectionné entre bicarbonate de sodium et bicarbonate de potassium, et où la concentration du sel de bicarbonate est comprise entre 0,1 g/l et 10 g/l de la composition totale dans le cas du bicarbonate de sodium, et est comprise entre 0,1 g/l et 20 g/l de la composition totale dans le cas du bicarbonate de potassium.

7. Composition de lavage intestinal destinée à l'usage selon la revendication 1, où la composition comprend en outre un anti-inflammatoire gastro-intestinal sélectionné entre gingembre, menthe poivrée et camomille, et où la concentration dudit anti-inflammatoire gastro-intestinal est comprise entre 5 g/l et 50 g/l de la composition totale.

8. Composition de lavage intestinal destinée à l'usage selon la revendication 1, où le premier ingrédient de lavage est le xylitol.

9. Composition de lavage intestinal destinée à l'usage selon la revendication 1, où le premier ingrédient de lavage est le sorbitol dans le cas du bicarbonate de potassium.

10. Composition de lavage intestinal destinée à l'usage selon la revendication 1, où le premier ingrédient de lavage est un mélange de xylitol et de sorbitol.

11. Composition de lavage intestinal destinée à l'usage selon la revendication 1, où, quand le deuxième ingrédient de lavage est un mélange d'acide ascorbique et d'un sel d'acide ascorbique, le rapport de masse entre l'acide ascorbique et le sel d'acide ascorbique est compris entre 99:1 et 60:40.

12. Composition de lavage intestinal destinée à l'usage selon la revendication 1, où le solvant aqueux est de l'eau, de l'eau gazéifiée, de l'eau ionisée alcaline ou une boisson.

13. Composition de lavage intestinal destinée à l'usage selon la revendication 1, où le solvant aqueux est de l'eau gazéifiée.

14. Composition de lavage intestinal destinée à l'usage selon la revendication 1, où la concentration du premier ingrédient de lavage est comprise entre 50 g/l et 150 g/l de la composition totale.

15. Composition de lavage intestinal destinée à l'usage selon la revendication 1, où la concentration du deuxième ingrédient de lavage est comprise entre 30 g/l et 200 g/l de la composition totale.

16. Composition de lavage intestinal destinée à l'usage selon la revendication 1, où le volume du solvant aqueux est compris entre 0,3 1 et 0,07 l.

17. Composition de lavage intestinal destinée à l'usage selon l'une des revendications 1 à 16, où la préparation est un produit en une seule partie.

18. Composition de lavage intestinal destinée à l'usage selon l'une des revendications 1 à 16, où la préparation est un produit en deux parties.

19. Composition de lavage intestinal destinée à l'usage selon la revendication 18, où la préparation est formulée comme produit en deux parties consistant en (i) un emballage contenant une solution fortement concentrée formulée par mélange de tous les ingrédients dans 0,05 1 à 0,2 l du volume total du solvant aqueux et (ii) un emballage distinct contenant le reste du solvant aqueux, équivalent à son volume total moins le volume utilisé pour formuler la solution fortement concentrée.
